# EUROPEAN PATENT APPLICATION

(11) **EP 4 339 741 A1**
(43) Date of publication of application: **20.03.2024**
(21) Application number: 22807555.2
(22) Date of filing: 13.05.2022
(51) Int. Cl.: G06F 3/01, G06F 3/0481, G06Q 50/10, G09B 9/00

(54) **ELECTRICAL CONDITION DATA MANAGEMENT DEVICE THAT CAUSES PERCEPTION OF FLAVOR TO CHANGE**

(30) Priority: 14.05.2021 JP 2021082429
(71) Applicant: AJINOMOTO CO., INC., Chuo-ku Tokyo 104-8315 (JP)
(72) Inventor: MATSUMOTO, Ryo, Kawasaki-shi, Kanagawa 210-8681 (JP); SATO, Kazuma, Kawasaki-shi, Kanagawa 210-8681 (JP)
(74) Representative: Strehl Schübel-Hopf & Partner
(86) International application number: PCT/JP2022/020192
(87) International publication number: WO 2022/239857

(57) **Abstract**

An object of the present invention is to provide an electrical condition data management apparatus, a program, and a method capable of modifying a taste to one's own preference or generating a taste to one's own preference. In the present embodiment, 1) electrical condition data that changes a feeling of a taste is read out from a storage unit, 2) the read electrical condition data is transmitted to an instrument capable of causing electricity to flow through a human body, 3) a user inputs an impression of the taste when the user ingests an actual food in a state where the electricity corresponding to the transmitted electrical condition data flows through the user via the instrument, and 4) the input impression is reflected in the electrical condition data stored in the storage unit.

## Description

### TECHNICAL FIELD

The present invention relates to an electrical condition data management apparatus, a program, and a method.

### BACKGROUND ART

Patent Document 1 discloses a food taste reproduction system capable of easily reproducing the taste of food based on taste data. Patent Document 2 discloses a system that generates a taste profile model.

### PRIOR ART REFERENCES

### PATENT DOCUMENTS

Patent Document 1: Japanese Patent 6,644,591
Patent Document 2: US 2017/0,011,145 A

### DISCLOSURE OF INVENTION

### PROBLEM TO BE SOLVED BY THE INVENTION

However, a technique capable of modifying a taste to one's own preference or generating a taste to one's own preference has not been previously developed.

The present invention has been made in view of the problems, and an object of the present invention is to provide an electrical condition data management apparatus, a program, and a method capable of modifying a taste to one's own preference or generating a taste to one's own preference.

### MEANS FOR SOLVING PROBLEM

In order to solve the problem and achieve the object, an electrical condition data management apparatus according to one aspect of the present disclosure includes a control unit, a storage unit, and a communication unit. The storage unit stores electrical condition data that changes a feeling of taste. The control unit includes a reading-out unit that reads out the electrical condition data from the storage unit, a first transmission unit that transmits the electrical condition data read out by the reading-out unit to an instrument including a communication unit, the instrument being capable of generating electricity corresponding to the electrical condition data received via the communication unit and causing the generated electricity to flow through a human body, an input unit that inputs an impression of a taste held by a user in a state where the electricity corresponding to the electrical condition data transmitted by the first transmission unit flows through the user via the instrument, and a reflection unit that reflects the impression input by the input unit in the electrical condition data stored in the storage unit.

The electrical condition data may be determined based on biological data.

The biological data may be activation data of an *in vivo* protein used when an organism feels the taste.

The *in vivo* protein may be a receptor or an ion channel.

The numerical value of the electrical condition data may be a numerical value corresponding to an intensity of the electrical condition data required to activate, even when a certain substance is not provided to the *in vivo* protein, the *in vivo* protein to the same extent as when the certain substance is provided to the *in vivo* protein.

The storage unit may further store at least one of personal information of the user, instrument information of the instrument, food information of food, and environmental information of an intake environment, and the reflection unit may further reflect the at least one information in the electrical condition data stored in the storage unit.

The reflection unit may input the electrical condition data after the at least one information corresponding to the user and the impression are reflected to a machine learning model that outputs, when the at least one information and the electrical condition data are input to the reflection unit, a congeniality degree between the at least one information and the electrical condition data, and may determine, when the machine learning model outputs a result indicating that the congeniality degree therebetween is good, that the electrical condition data after the impression is reflected also reflect the information.

The control unit may further include a generation unit that generates the electrical condition data, and a registration unit that registers, in the storage unit, the electrical condition data generated by the generation unit.

The storage unit may further store a plurality of templates related to the electrical condition data, and the generation unit may cause the user to generate the electrical condition data by causing the user to select a desired template from among the templates and causing the user to rewrite the selected template.

The control unit may further include a second transmission unit that transmits the electrical condition data to another electrical condition data management apparatus.

A plurality of pieces of the electrical condition data may be stored in the storage unit, the control unit may further include an acquisition unit that acquires preference information of the user, and a determination unit that determines, based on the preference information acquired by the acquisition unit, the electrical condition data matching a preference of the user from among the pieces of electrical condition data stored in the storage unit, and the reading-out unit may read out, from the storage unit, the electrical condition data determined by the determination unit.

The storage unit may further store classification data including a plurality of classifications of the food, the electrical condition data in the storage unit may be stored in association with the classification of the food, the control unit may further include a selection unit that causes the user to select the classification of the food, the reading-out unit may read out, from the storage unit, the electrical condition data associated with the classification selected by the selection unit, and the input unit may input the impression of the taste when the user ingests an actual food corresponding to the selected classification in the state.

The electrical condition data management apparatus according one aspect of the present disclosure may further include an imaging unit, the electrical condition data in the storage unit may be stored in association with the classification of the food, the control unit may include a reading unit that reads a figure pattern from an image obtained by imaging, by the imaging unit, a subject on which the figure pattern in which the classification of the food is recorded is displayed and from which the classification of the food is visually recognizable, the reading-out unit may read out, from the storage unit, the electrical condition data associated with the classification recorded in the figure pattern read by the reading unit, and the input unit may input the impression of the taste when the user ingests an actual food corresponding to the classification visually recognized from the subject in the state.

The storage unit may further store expression data including a plurality of expressions related to the taste, and the input unit may cause the user to select the appropriate expression from among the expressions.

The electrical condition data may change the feeling of the taste.

The electrical condition data may include at least one of a numerical value of an intensity of the electricity, a numerical value of a frequency of the electricity, and a numerical value of a duty ratio.

A program according to one aspect of the present disclosure is a program for causing a control unit of an electrical condition data management apparatus including the control unit, a storage unit that stores electrical condition data that changes a feeling of a taste, and a communication unit to function as a reading-out unit that reads out the electrical condition data from the storage unit, a first transmission unit that transmits the electrical condition data read out by the reading-out unit to an instrument including a communication unit, the instrument being capable of generating electricity corresponding to the electrical condition data received via the communication unit and causing the generated electricity to flow through a human body, an input unit that inputs an impression of a taste held by a user in a state where the electricity corresponding to the electrical condition data transmitted by the first transmission unit flows through the user via the instrument, and a reflection unit that reflects the impression input by the input unit in the electrical condition data stored in the storage unit.

A method according to one aspect of the present disclosure is a method executed by a control unit of an electrical condition data management apparatus including the control unit, a storage unit that stores electrical condition data that changes a feeling of a taste, and a communication unit, the method including a step of reading out the electrical condition data from the storage unit, a step of transmitting the read electrical condition data to an instrument including a communication unit, the instrument being capable of generating electricity corresponding to the electrical condition data received via the communication unit and causing the generated electricity to flow through a human body, a step of inputting an impression of a taste held by a user in a state where the electricity corresponding to the transmitted electrical condition data flows through the user via the instrument, and a step of reflecting the input impression in the electrical condition data stored in the storage unit.

### EFFECT OF THE INVENTION

According to the present invention, there is an effect that a taste can be modified to one's own preference or a taste can be generated to one's own preference.

### BRIEF DESCRIPTION OF DRAWINGS

FIG. 1 is a block diagram showing an example of a configuration of a system 1;
FIG. 2 is a block diagram showing an example of a configuration of an electrical condition data management apparatus 10;
FIG. 3 is a block diagram showing an example of a configuration of an instrument 12;
FIG. 4 is a diagram for conceptually explaining an example of processing executed by the system 1; and
FIG. 5 is a graph for explaining a relationship between a compound concentration and an activity intensity of a receptor, and a graph for explaining an example of an electrical intensity variable rate of electrical condition data.

### BEST MODES FOR CARRYING OUT THE INVENTION

Hereinafter, embodiments of an electrical condition data management apparatus, a program, and a method according to the present invention will be explained in detail with reference to the drawings. It is noted that the present invention is not limited by the present embodiments.

### 1. Configuration

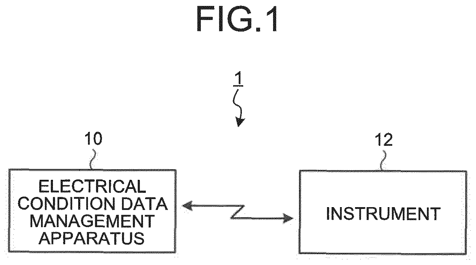

FIG. 1 is a block diagram that depicts an example of a configuration of a system 1. The system 1 includes an electrical condition data management apparatus 10 and an instrument 12. In the present embodiment, it is assumed that a certain user (hereinafter, referred to as a "user X".) uses both the electrical condition data management apparatus 10 and the instrument 12, but the present invention is not to be construed as being limited to such assumption.

The electrical condition data management apparatus 10 is constructed based on, for example, a commercially available portable or a stationary information processing apparatus (for example, a smartphone, a wearable terminal, a tablet terminal, a notebook personal computer, and a desktop personal computer). The instrument 12 is an instrument that allows electricity to flow through the user X through a portion of the user X (for example, a portion around the oral cavity (for example, cheeks, jaws, or lips), a portion in the oral cavity (for example, tongue), an ear, a neck, and a wrist).

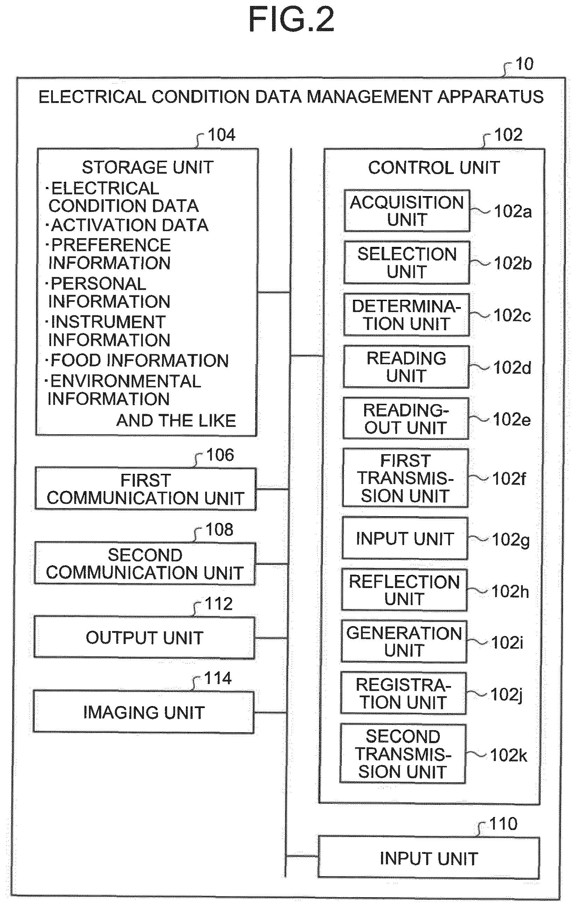

FIG. 2 is a block diagram that depicts an example of a configuration of the electrical condition data management apparatus 10. The electrical condition data management apparatus 10 includes a control unit 102, a storage unit 104, a first communication unit 106, a second communication unit 108, an input unit 110, an output unit 112, and an imaging unit 114. The respective units included in the electrical condition data management apparatus 10 are communicably connected to each other via any communication path.

The first communication unit 106 is a device conforming to a short-range wireless communication standard such as Bluetooth (registered trademark). The second communication unit 108 is a device capable of performing wired or wireless communication via a network (for example, a local area network (LAN), and the Internet) and conforming to a communication standard different from the communication standard of the first communication unit 106. The input unit 110 is a device that inputs information, such as a keyboard, a mouse, a microphone, a monitor that implements a pointing device function in cooperation with the mouse, or a touch panel. The output unit 112 is a device that outputs information, such as a monitor, a touch panel, or a speaker. The imaging unit 114 is a device that images a subject and generates an image, such as a camera.

The storage unit 104 is a device that stores various databases, tables, files, or the like. As the storage unit 104, for example, a memory device such as a random access memory (RAM) or a read only memory (ROM), a fixed disk device such as a hard disk, a flexible disk, an optical disk, or the like may be used. A computer program for giving instructions to a central processing unit (CPU) or a graphics processing unit (GPU) to perform various types of processing in cooperation with an operating system (OS) may be recorded in the storage unit 104.

The storage unit 104 stores electrical condition data that changes a feeling of taste (for example, a basic taste (a sweet taste, a sour taste, a salty taste, a bitter taste, and palatability), and a taste such as richness, a fatty feeling, or a spice feeling) of a food (all the foods and drinks except a pharmaceutical product and a quasi-pharmaceutical product specified in the Pharmaceutical Affairs Law).

The electrical condition data may include, for example, at least one of a numerical value of the intensity of electricity, a numerical value of the frequency of electricity, and a numerical value of the duty ratio. Here, for example, Non-Patent Document 1 "Augmented Flavours: Modulation of Flavour Experiences Through Electric Taste Augmentation, Nimesha Ranasinghe, National University of Singapore, Food Research International, 117 (2019) 60-68", Non-Patent Document 2 "Electrical Stimulation Technique with Taste Substance Selectivity, Naoya Hara et al., the 25th Virtual Reality Society of Japan Convention Paper (September 2020)", and Non-Patent Document 3 "Continuous Enhancement of Five Tastes by Continuous Rectangular Waveform Current Stimulation, Akiyoshi Hara, Journal of Virtual Reality Society of Japan Vol. 24, No. 1, 2019" can be referred to for the fact that a feeling of taste can be changed by the frequency and the duty ratio.

In addition, for example, a plurality of pieces of electrical condition data may be stored in the storage unit 104. Furthermore, the electrical condition data in the storage unit 104 may be stored in association with, for example, a classification of food (for example, a general name of food).

The electrical condition data may be determined based on, for example, biological data. The biological data is, for example, activation data of an *in vivo* protein (a receptor, an ion channel, or the like) used when an organism feels taste. By using the biological data, data closer to the truth (= closer to the taste actually felt by a person) can be collected as compared with a case in which physical data or mathematical data is used (for example, when a sensor is used or when mathematical expression is used), and as such a more precise value can be determined as the electrical condition data.

The storage unit 104 may further store, for example, the activation data. The activation data is, for example, receptor-ion channel activation data such as a sweet taste receptor T1R2/T1R3, an umami taste receptor T1R1/T1R3, a bitter taste receptor T2Rs, a capsaicin receptor TRPV1, and a calcium sensing receptor CaSR. The activation data includes, for example, activation patterns of various receptor/ion channels for a solution containing a sweet taste component, a food extract, and the like, and the electrical condition data for reproducing a taste of a food can be designed based on the activation patterns. As a method of obtaining this activation pattern, it is possible to use a method of evaluating the activation of various receptor/ion channels such as animal cells, yeast, biosensors coated with receptor/ion channel protein/taste cell/tongue tissue, liposomes, vesicles, lipid bilayers, cell membranes, and taste sensors.

Examples of the receptor include TAS1R1, TAS1R2, TAS1R3, CASR, TAS2R1, TAS2R3, TAS2R4, TAS2R5, TAS2R7, TAS2R8, TAS2R9, TAS2R10, TAS2R13, TAS2R14, TAS2R16, TAS2R19, TAS2R20, TAS2R30, TAS2R31, TAS2R38, TAS2R39, TAS2R40, TAS2R41, TAS2R42, TAS2R43, TAS2R45, TAS2R46, TAS2R50, TAS2R60, and FFAR4 respectively indicated by gene names.

Examples of the ion channel include SCNN1A, SCNN1B, SCNN1G, SCNN1D, OTOP1, OTOP2, OTOP3, TRPV1, TRPV2, TRPV3, TRPV4, TRPV5, TRPV6, TRPA1, TRPM8, TRPC1, TRPC2, TRPC3, TRPC4, TRPC5, TRPC6, TRPC7, TRPM1, TRPM2, TRPM3, TRPM4, TRPM5, TRPM6, TRPM7, TRPM8, TRPML1, TRPML2, TRPML3, TRPP1, TRPP2, and TRPP3 respectively indicated by gene names.

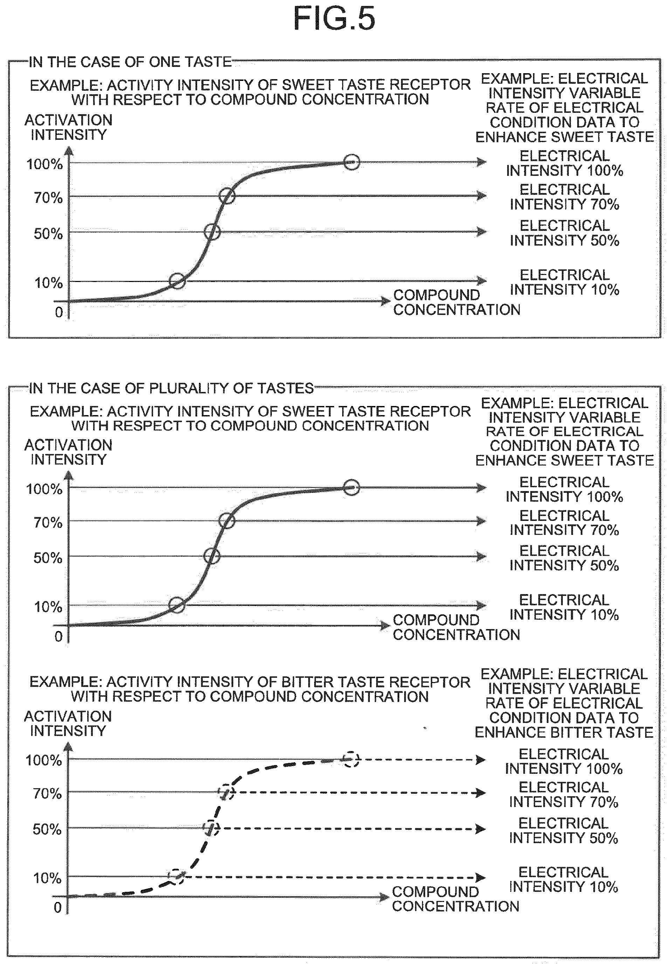

The way the electrical condition data is determined based on the activation data will be explained. The electrical condition data may be determined from an activation intensity of a receptor or an ion channel. The receptor and the ion channel transmit signals into a cell by a compound (an agonist, an antagonist, and the like) that binds to the receptor and the ion channel. When the signals are further transmitted to the nerve and the brain, an organism feels taste, smell, temperature, and the like. These signals can be evaluated, for example, by measuring an increase or decrease in intracellular calcium concentration, a change in cell membrane potential, and the like. The activation intensity of the receptor or the ion channel depends on the concentration of the compound to be evaluated, and generally draws a sigmoid curve. Therefore, for example, when the maximum activity value of the sigmoid curve indicating the activation intensity of the sweet taste receptor T1R2/T1R3 is 100%, the electrical condition data can be varied depending on the activation intensity. For example, as illustrated in FIG. 5, when the activation intensity of the receptor or the ion channel is 50%, it is possible to automatically design the electrical condition data corresponding to the activation intensity by associating the activation intensity such that the intensity of the electrical condition is variable to 50%.

In other words, the numerical value of the electrical condition data is, for example, a numerical value corresponding to the intensity of the electrical condition data necessary for activating the *in vivo* protein to the same extent as when a certain substance is provided to the *in vivo* protein even when the *in vivo* protein is not provided with the certain substance.

In the case of a plurality of tastes (for example, a sweet taste and a bitter taste), it is possible to cope with the tastes by increasing the number of electric circuits provided in the instrument and increasing the number of energized portions. For example, when it is desired to adjust two tastes at the same time, the tastes can be adjusted by preparing a bifurcated fork and providing different electrical conditions to the respective tips. Alternatively, the tastes can be adjusted by reflecting one type of electrical condition data on the fork and reflecting the other type of electrical condition data on the bracelet. It is noted that the content explained herein is merely an example, and does not limit the type of receptor/ion channel or the type/number of instruments to be used to be reflected in the electrical condition data.

Furthermore, the storage unit 104 may further store, for example,-preference information of the user X. It is noted that examples of the preference information include 1) a questionnaire survey result regarding preference, 2) preference of the strength of taste that changes depending on a physical condition, a mood, a meal environment, or the like on that day, and 3) taste preference based on a religious, ideological, or cultural view. For example, the storage unit 104 may further store classification data including a plurality of food classifications. Furthermore, the storage unit 104 may further store expression data including, for example, a plurality of expressions (for example, "optimal, strong, light" or "good, normal, bad") related to the taste. The storage unit 104 may further store template data including a plurality of templates related to the electrical condition data.

Furthermore, the storage unit 104 may further store, for example, at least one of personal information on the user X, instrument information on the instrument 12, food information on food, or,environmental information on an intake environment of food.

Here, examples of the personal information include a physical condition, a medical history, an ingestion history, a sex, a race, an age, gene data, a preference, blood data, a pulse, eating habit data, a heart rate, a blood pressure, a stress level, an intestinal flora, an oral environment, a meal environment (for example, home or dining out), a religion, a thought, a culture, a hunger/satiety degree, a muscle mass, a body mass index (BMI), a physical/mental fatigue degree, allergy, a body shape, a blood glucose level, electrical resistance of a human body, and a smoking history.

Examples of the instrument information include an amount of current, a frequency, a duty ratio, a shape, a material, and a component of an instrument (for example, presence or absence of the component). It is noted that examples of the shape include a spoon, a fork, a chopstick, a bowl, a beer glass, a cocktail glass, a cup, a renge spoon, a toothpick, a flat dish, a tea cup, a knife, a spreader, a rice paddle, a ladle, a cigarette, a wine glass, a champagne glass, a rock glass, a shot glass, a tumbler, a goblet, an amuse glass, a piercing, a mouthpiece, a choke, an implant, a necklace, a wristband, a headband, a helmet, glasses, a mask, a headgear, an earphone, a headphone, a neck warmer, a wristwatch, a denture, a post crown, an implant, an earring, a ring, a bracelet, a collar, a choker, a seal, a thin film sensor, a wearable sensor, a clothing, a contact lens, a toupee, a wig, a tiara, a tie, a virtual reality (VR)/augmented reality (AR)/mixed reality (MR) device, a brain-machine interface (skull attachment and intracerebral implantation), a chair, a gaming chair, a desk, a table, a glove, a shoe, a sock, and the like. That is, the instrument may be a mounting tool to be mounted on the human body or a contact tool to be in contact with the human body. Examples of the material include metal (for example, gold, silver, copper, and iron), wood, glass, plastic, rubber, and conductive resin. In addition, examples of the components include a wireless connection function, a nutrient/temperature detection function, an electrical condition automatic presentation function (an artificial intelligence (AI) function), an electrical intensity adjustment function, a temperature control function, a vibration function, and the like.

In addition, examples of the food information include a meal menu (for example, salad, gyoza, fried rice, and mapo bean curd), elasticity, viscosity, a water content, various nutrients, a temperature at the time of eating, a material used, a production area of a material, a taste/odor component, a texture, an amino acid ratio, a vitamin ratio, a color of a meal, a pH of a meal, and the like.

Furthermore, examples of the environmental information include a dining place (for example, home or store), a contact form with a person at the time of eating (for example, face-to-face or online), the number of persons at the time of eating (for example, one or two persons), music in a meal environment (for example, genre or tempo), a temperature and humidity of a meal environment, and a smell of the meal environment (for example, a numerical value representing a smell or expression of a smell).

The control unit 102 is a CPU, a GPU, or the like that integrally controls the electrical condition data management apparatus 10. The control unit 102 includes an internal memory that stores a control program such as an OS, a program defining various processing procedures, or required data, and executes various types of information processing based on these programs.

The control unit 102 functionally and conceptually includes an acquisition unit 102a, a selection unit 102b, a determination unit 102c, a reading unit 102d, a reading-out unit 102e, a first transmission unit 102f, an input unit 102g, a reflection unit 102h, a generation unit 102i, a registration unit 102j, and a second transmission unit 102k.

The acquisition unit 102a is a unit that acquires the preference information of the user X.

The selection unit 102b is a unit that selects the classification of food. For example, the selection unit 102b may cause the user X to select the classification from among a plurality of classifications of foods included in the classification data stored in the storage unit 104.

The determination unit 102c is a unit that determines, based on the preference information acquired by the acquisition unit 102a, electrical condition data (for example, electrical condition data optimum for the preference of the user X) that matches the preference of the user X from among the pieces of electrical condition data stored in the storage unit 104. For example, when the electrical condition data corresponding to a light taste, the electrical condition data corresponding to a strong taste, and the electrical condition data corresponding to strength of a standard taste are stored in the storage unit 104, and the user X prefers the strong taste, the determination unit 102c may select the electrical condition data corresponding to the strong taste. It is noted that, for example, the determination unit 102c may determine, based on the preference information acquired by the acquisition unit 102a and the classification selected by the selection unit 102b, the electrical condition data associated with the selected classification and matching the preference of the user X from among the pieces of electrical condition data stored in the storage unit 104.

The reading unit 102d is a unit that reads a figure pattern from an image obtained by imaging, by the imaging unit 114, a subject (for example, a food package and a menu table) on which the figure pattern (for example, a one-dimensional bar code, and a two-dimensional bar code such as a QR code (registered trademark)) in which the classification of the food is recorded is displayed and from which the classification of the food is visually recognizable.

The reading-out unit 102e is a unit that reads out the electrical condition data from the storage unit 104. For example, the reading-out unit 102e may read out the electrical condition data associated with the classification selected by the selection unit 102b from the storage unit 104. The reading-out unit 102e may read, for example, the electrical condition data determined by the determination unit 102c from the storage unit 104. In addition, for example, the reading-out unit 102e may read, from the storage unit 104, the electrical condition data associated with the classification recorded in the figure pattern read by the reading unit 102d.

The first transmission unit 102f is a unit that transmits the electrical condition data read by the reading-out unit 102e to the instrument 12 via the first communication unit 106.

The input unit 102g is a unit that inputs an impression (for example, an impression of a taste when actual food is ingested) of a taste held by the user X in a state where electricity corresponding to the electrical condition data transmitted by the first transmission unit 102f is flowing through the user X via the instrument 12 (hereinafter, referred to as an "ON state".).

It is noted that, for example, the input unit 102g may cause the user X to input the impression of the taste held by the user X in the ON state via the input unit 110. Furthermore, for example, the input unit 102g may cause the user X to input, via the input unit 110, the impression of the taste when the user X ingests the actual food corresponding to the classification selected by the selection unit 102b in the ON state.

Furthermore, for example, the input unit 102g may cause the user X to input, via the input unit 110, the impression of the taste when the user X ingests the actual food corresponding to the classification visually recognized by the user X from the subject in the ON state. Furthermore, for example, the input unit 102g may cause the user X to select, via the input unit 110, an appropriate expression from a plurality of expressions related to the taste included in the expression data stored in the storage unit 104.

The reflection unit 102h is a unit that reflects the impression input by the input unit 102g in the electrical condition data stored in the storage unit 104. It is noted that the reflection unit 102h may reflect the impression input by the input unit 102g in the electrical condition data to be read by the reading-out unit 102e in the storage unit 104. Specifically, the reflection unit 102h may update the electrical condition data to be read to data corresponding to the impression. More specifically, the reflection unit 102h may update the electrical condition data according to a preset way of changing the electrical condition data according to the impression (for example, an increase/decrease width of a numerical value) (hereinafter, referred to as a "first changing method".). For example, as the first changing method, when a relationship between the expressions related to the taste and a change amount of the numerical value in the electrical condition data is set such that ""light", "+1.0"", ""slightly light", "+0.1"", ""appropriate", "0.0"", ""slightly strong", "-0.1"", and ""strong", "-1.0"", and an adjustment range of the numerical value in the electrical condition data (for example, a range of one or more and five or less) is set, the reflection unit 102h may add the numerical value associated with the expression selected by the user X to the numerical value in the electrical condition data on condition that the numerical value falls within the adjustment range. Furthermore, the reflection unit 102h may cause the user X to directly adjust the electrical condition data to be read out in the reading-out unit 102e in the storage unit 104. For example, the reflection unit 102h may display, on the output unit 112, a screen (not illustrated) on which the numerical value in the electrical condition data is adjustable stepwise or continuously within a predetermined range (for example, a range of one or more and five or less) by a predetermined operation (for example, a button operation or a slider bar operation), and cause the user X to directly adjust, via the screen, the numerical value in the electrical condition data.

Furthermore, for example, the reflection unit 102h may reflect at least one of the personal information on the user X, the instrument information on the instrument 12, the food information (specifically, the food information corresponding to the classification selected by the selection unit 102b or the food information corresponding to the classification recorded in the figure pattern read by reading unit 102d) of the food ingested by the user X, and the environmental information at the time of ingesting the food of the user X (hereinafter, referred to as "user data".) in the electrical condition data to be read out by the reading-out unit 102e in the storage unit 104.

Furthermore, for example, the reflection unit 102h may reflect the user data in the electrical condition data after the impression is reflected. Furthermore, for example, the reflection unit 102h may update the electrical condition data after the impression is reflected to one corresponding to the user data.

Furthermore, for example, the reflection unit 102h may input the electrical condition data after the user data and the impression are reflected in a machine learning model that outputs a congeniality degree (for example, whether congeniality is good or bad) of the user data and the electrical condition data when the user data and the electrical condition data are input, and determine that the electrical condition data after the impression is reflected also reflects the user data when a result indicating that the congeniality degree is good is output from the machine learning model. It is noted that the machine learning model may be obtained, for example, by performing machine learning by a predetermined method (for example, partial least squares (PLS), random forest (RF), extremely randomized trees (ERT), least absolute shrinkage and selection operator (LASSO), ridge, elastic net (EN), linear support vector regression (LSVR), non-linear support vector regression (NLSVR), gaussian process (GP), gene algorithm-based partial least squares (GA_PLS), adaptive boosting (AdaBoost), extreme gradient boosting (XGBoost), deep neural network (DNN), or the like) based on learning data (input data: user data and electrical condition data, output data: degree of congeniality degree) prepared in advance. As the learning data, for example, a result of congeniality indicating good or bad, the result being obtained by verifying a change of the feeling of the taste when a taste panelist ingests food X with respect to a plurality of pieces of electrical condition data, may be used.

The generation unit 102i is a unit that generates the electrical condition data. For example, the generation unit 102i may cause the user X to generate the desired electrical condition data via the input unit 110. In addition, for example, the generation unit 102i may cause the user X to select a desired template from a plurality of templates included in the template data stored in the storage unit 104 via the input unit 110, and cause the user X to rewrite the selected template via the input unit 110. Thereby, the original electrical condition data of the user X can be generated.

The registration unit 102j is a unit that registers the electrical condition data generated by the generation unit 102i in the storage unit 104. For example, the registration unit 102j may register the generated electrical condition data in the storage unit 104 in association with the classification of the food.

The second transmission unit 102k is a unit that transmits the electrical condition data in the storage unit 104 to the other electrical condition data management apparatus 10 via the second communication unit 108. As a result, for example, the electrical condition data management apparatus 10 and the other user having the instrument 12 can share the electrical condition data.

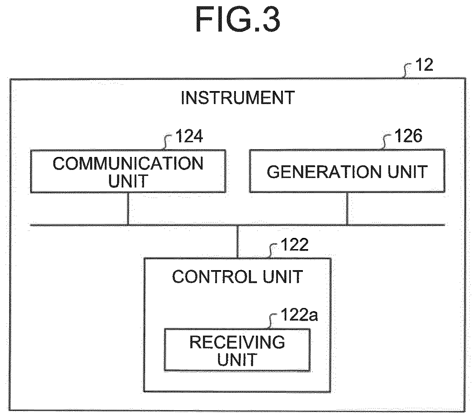

FIG. 3 is a block diagram showing an example of a configuration of the instrument 12. The instrument 12 includes at least a control unit 122, a communication unit 124, a generation unit 126, and hardware (not illustrated) that causes electricity generated by the generation unit 126 to flow through a human body through a human body portion (for example, a portion around the oral cavity (for example, cheeks, jaws, and lips), a portion in the oral cavity (for example, tongue), an ear, a neck, and a wrist). The control unit 122 is a device that integrally controls the instrument 12, and includes a receiving unit 122a. The receiving unit 122a is a unit that receives the electrical condition data transmitted from the information processing apparatus 10 via the communication unit 124. The control unit 122 transfers the electrical condition data received by the receiving unit 122a to the generation unit 126. The communication unit 124 is a device conforming to a short-range wireless communication standard such as Bluetooth (registered trademark). The generation unit 126 is a device that generates electricity according to the electrical condition data transferred from the control unit 122. It is noted that, as the hardware configuration of the instrument 12, for example, JP-T-2007-521868, JP-A-2018-42991, JP-A-2019-212799, JP-A-2021-045399, Non-Patent Document 1, Non-Patent Document 4 "Digitally Stimulating the Sensation of Taste Through Electrical and Thermal Stimulation, Nimesha Ranasinghe, National University of Singapore, 23-Aug-2012", and the like may be referred to. Furthermore, the instrument 12 may further have any one or both of a hardware configuration and a software configuration to cause the user X to directly adjust the electrical condition data.

### 2. Processing

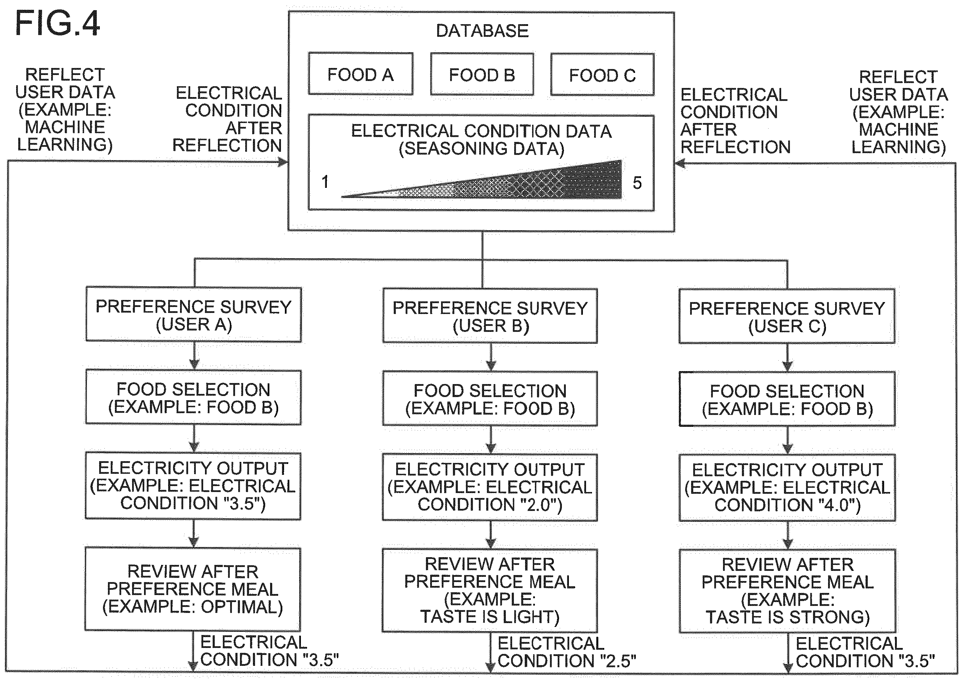

FIG. 4 is a diagram for conceptually explaining an example of processing executed by the system 1. It is noted that the processing explained in FIG. 4 is merely an example, and the present invention is not to be construed as being limited to the processing.

### Step S1

First, the acquisition unit 102a acquires, from the storage unit 104, preference information (for example, preference of strength of the taste) of the user X (corresponding to a user A, a user B, or a user C explained in FIG. 4). It is noted that the acquisition of the preference information may be skipped.

### Step S2

Next, the selection unit 102b causes the user X to select, via the input unit 110, the classification of the food to be eaten by the user X using the instrument 12 in the ON state from among the classifications of the food included in the classification data. FIG. 4 explains, as an example, that the user X selects food B.

### Step S3

Next, the determination unit 102c determines, from among the pieces of electrical condition data stored in the storage unit 104, the electrical condition data optimum for the preference of the user X associated with the selected classification based on the preference information acquired in step S1 and the classification selected in step S2. FIG. 4 explains that, when the user A who likes a taste of a standard strength selects the food B, an optimal electrical condition for the user A is determined to be "3.5", and when the user B who likes a light taste selects the food B, an optimal electrical condition for the user B is determined to be "2.0", and when the user C who likes a strong taste selects the food B, an optimal electrical condition for the user C is determined to be "4.0".

Next, the reading-out unit 102e reads out the determined electrical condition data from the storage unit 104.

Next, the first transmission unit 102f transmits the read electrical condition data to the instrument 12 via the first communication unit 106.

Next, the receiving unit 122a receives the electrical condition data transmitted from the information processing apparatus 10 via the communication unit 124.

Next, the control unit 122 transfers the received electrical condition data to the generation unit 126.

Next, the generation unit 126 generates electricity according to the transferred electrical condition data.

At this stage, the ON state is established. Then, in the ON state, the user X ingests the actual food corresponding to the classification selected in step S2. Then, the user X operates, for example, the electrical condition data management apparatus 10 to activate the input unit 102g after the end of the ingestion (after the meal) .

### Step S4

Next, the input unit 102g causes the user X to select the expression of the taste closest to the impression of the taste when the actual food is ingested in the ON state from among the expressions related to the taste included in the expression data stored in the storage unit 104. It is noted that the impression of the taste may be, for example, an impression narrowed down to five basic tastes and other taste sensations, an impression including temporal information on a feeling of a taste, an impression including a taste expression used at the time of expressing the taste, or the like. Furthermore, a method of inputting the impression of the taste may be, for example, a numerical input, a character input using typing on a PC or a flick input on a smartphone, a voice input using a voice recognition function, a line-of-sight input by eye movement recognition, a brain wave input by brain wave reading, or any combination thereof. Here, examples of the impressions narrowed down to the five basic tastes and other taste sensations include strong or light, salty (a salty taste is strong) or not salty (a salty taste is weak), sweet (a sweet taste is strong) or not sweet (a sweet taste is weak), sour (a sour taste is strong) or not sour (a sour taste is weak), bitter (a bitter taste is strong) or not bitter (a bitter taste is weak), strong umami or weak umami, spicy or not spicy, astringency or not astringency. Examples of the impression including the temporal information on the feeling of the taste include strong sweetness remaining after food is ingested, strong salty taste at the moment when food is put into the mouth, weak umami taste at the time when food spreads into the mouth, and strong bitterness at the moment when food is swallowed. Examples of the impression including the taste expression used at the time of expressing the taste include rich, salty, juicy, creamy, sweet and sour, kicky, spicy, plain, bittersweet, mellow, impactful, fruity, mild, and strong.

Next, the reflection unit 102h updates, based on the selected expression, the electrical condition data read in step S3 according to the first changing method (specifically, a relationship between the expressions related to the taste and a change amount of the numerical value in the electrical condition data). FIG. 4 explains that the electrical condition is maintained at "3.5" (the change amount is "0.0") when the user A selects "optimum", that the electrical condition is increased from "2.0" to "2.5" (the change amount is "+0.5") when the user B selects "taste is light", and that the electrical condition is decreased from "4.0" to "3.5" (the change amount is "-0.5") when the user C selects "taste is strong". That is, FIG. 4 explains that the electrical condition reflecting the impression of the taste of the user X is obtained.

Next, the reflection unit 102h inputs the user data stored in the storage unit 104 and the obtained electrical condition to the machine learning model, and determines that the obtained electrical condition also reflects the user data when a result indicating good congeniality is output from the machine learning model. FIG. 4 explains that the electrical condition (that is, the electrical condition personalized to the user X) further reflecting the user data is obtained.

Next, the reflection unit 102h stores the electrical condition data further reflecting the user data in the storage unit 104.

Through the above processing, the electrical condition data in the storage unit 104 is adjusted to match the preference of the user X.

### 3. Other embodiments

Although the embodiments of the present invention have been explained so far, the present invention may be implemented in various different embodiments other than the embodiments within the scope of the technical idea explained in the claims.

For example, among the respective types of processing explained in the embodiments, all or part of the processing explained as being automatically performed can be manually performed, or all or part of the processing explained as being manually performed can be automatically performed by a known method.

In addition, the processing procedure, the control procedure, the specific name, the information including the parameters such as the registration data and the search condition of each processing, the screen example, and the database configuration explained in the document and the drawings can be freely and selectively changed unless otherwise specified.

In addition, regarding each device, each explained component is functionally conceptual, and does not necessarily need to be physically configured as explained.

For example, all or any part of the processing functions included in the electrical condition data management apparatus 10, particularly each processing function performed by the control unit 102 may be implemented by a CPU or a GPU and a program interpreted and executed by the CPU or the GPU, or may be implemented as hardware by wired logic. It is noted that the program is recorded in a non-transitory computer-readable recording medium including programmed instructions for causing the information processing apparatus to execute the method according to the present invention, and is mechanically read by the information processing apparatus 10 as necessary. That is, in the storage unit 104 such as the ROM or the HDD, a computer program for giving instructions to the CPU or the GPU and performing various types of processing in cooperation with the OS is recorded. This computer program is executed by being loaded into the RAM, and configures a control unit in cooperation with the CPU or the GPU.

In addition, this computer program may be stored in an application program server connected to the electrical condition data management apparatus 10 via any network, and all or a part thereof can be downloaded as necessary.

The program according to the present disclosure may be stored in a non-transitory computer-readable recording medium, or may be configured as a program product. Here, the "recording medium" includes any "portable physical medium" such as a memory card, a USB memory, an SD card, a flexible disk, a magneto-optical disk, a ROM, an EPROM, an EEPROM, a CD-ROM, an MO, a DVD, and a Blu-ray (registered trademark) disc.

In addition, the "program" is a data processing method described in any language or description method, and may be in any format such as a source code or a binary code. It is noted that the "program" is not necessarily limited to a single program, and includes a program configured in a distributed manner as a plurality of modules or libraries, and a program that achieves its function in cooperation with a separate program represented by an OS. It is noted that a known configuration and procedure can be used for a specific configuration and a reading procedure for reading the recording medium, an installation procedure after reading, and the like in each device explained in the embodiment.

In addition, the electrical condition data management apparatus 10 may be configured as an information processing apparatus such as a known personal computer or a workstation, or may be configured as an information processing apparatus to which any peripheral device is connected. In addition, the electrical condition data management apparatus 10 may be implemented by installing software (including a program, data, or the like) that implements the method of the present invention in the information processing apparatus.

Furthermore, a specific form of distribution and integration of the device is not limited to the explained form, and all or a part thereof can be functionally or physically distributed and integrated in any unit according to various additions or the like or according to a functional load. That is, the embodiments may be freely and selectively combined and implemented, or the embodiments may be selectively implemented.

In addition, by using the electrical condition data management apparatus, the program, and the method according to the present embodiment, it is possible to generate a taste that matches the user's preference of any food at any time and anywhere. Accordingly, the following applications can be performed.

### (1) Application to reduced salt diet

For example, a salt intake amount per day in renal disease patients (chronic renal disease patients and dialysis patients) is limited due to deterioration in renal function. However, lowering the amount of salt in the meal leads to deterioration in the taste of the food, and the patient cannot enjoy the meal more satisfactorily than before the disease. Accordingly, quality of life is greatly impaired. By utilizing the electrical condition data management apparatus, the program, and the method according to the present embodiment, the patient can set and design food having the flavor that suits the preference of the patient and eat the food even in a salt-restricted diet.

### (2) Application to space food

As compared with the earth, the amount of food and seasoning that can be stocked in space is more limited. Since transportation of food and seasoning to space requires enormous costs, it is important to suppress the transportation amount as much as possible, but if the transportation amount of food and seasoning is reduced, variations of "space food (menu, flavor, and the like)" are reduced. By utilizing the electrical condition data management apparatus, the program, and the method according to the present embodiment, it is possible to further expand the variations of the flavor of the space food and enhance the preference of the space food.

### (3) Application to personalized seasoning

Preference to meal changes depending on personal information such as an age, a sex, a culture, a food experience, a religion, a gene, and a physical condition. For example, there is a case in which "taste preference" is different even at home, and if it is attempted to prepare a meal according to the preference of each family member, it is necessary to separately prepare meals having different flavors, which takes time and effort. However, by utilizing the electrical condition data management apparatus, the program, and the method according to the present embodiment and individually adjusting the feeling of the taste by electricity, it is possible to enjoy the meal that suits the individual preference.

### (4) Application to game

The electrical condition data management apparatus, the program, and the method according to the present embodiment can also be applied to taste experience in a game. By outputting the electrical condition data to the user during the game, for example, it is possible to simulate the taste of a meal that an in-game character is eating. These electrical condition data can also be shared among players of the game. By fusing the experience of "taste" during the game, it is possible to further enhance a realistic feeling and a feeling of immersion in the game.

### (5) Application to social networking service (SNS)

"Taste transmission/reception" is enabled by posting the electrical condition data designed by the user on the SNS. The posted electrical condition data is downloaded to the followers of the user, and the followers who downloads the data can enjoy meals by utilizing the electrical condition data and share the electrical condition data with followers' acquaintances.

### (6) Application to online meal

Sharing the electrical condition data adjusting the taste online can evolve the experience during the online meal. For example, by matching the food to be eaten with a conversation partner online and sharing the electrical condition data designed by the partner online, it is possible to feel the same taste as the meal that the partner is eating.

### (7) Application to preference visualization

By accumulating data indicating what kind of electrical condition data a user utilizes, the preference for each user can be visualized. For example, when eating a commercially available gyoza product X, it is possible to categorize the user's preference by acquiring information such as the user A who uses the electrical condition data for increasing the salty taste 1.3 times and the user B who uses the electrical condition data for increasing the umami taste 1.2 times. By providing this classification result to the user, the user can understand his/her own preference, and can more easily select a product that matches him/her.

### (8) Application to video content

By utilizing the electrical condition data management apparatus, the program, and the method according to the present embodiment, the taste (the electrical condition data) linked with a video content can be provided. For example, it is possible for a user to feel the taste of food eaten by a character in real time while watching a movie. It is also possible to transmit a taste corresponding to a specific scene in the video content to the user. For example, by sending, to the user, the electrical condition data suitable for a scene, such as a "sour taste" in a scene where a character is broken-hearted, and a "bitter taste" when a character is attacked by an enemy, the user can enhance a feeling of immersion and a realistic feeling in the video content.

### (9) Application to palatable content

By utilizing the electrical condition data management apparatus, the program, and the method according to the present embodiment, it is possible to generate a taste corresponding to the four senses including a visual sense, an auditory sense, a tactile sense, and an olfactory sense. For example, when listening to classical music, it is possible to provide a new experience of enjoying a taste while enjoying music by continuously distributing the electrical condition data (the electrical condition data exhibiting a sour taste for a high sound, the electrical condition data exhibiting a bitter taste for a low sound, and the like) corresponding to a flowing sound. In addition, when the electrical condition data is designed according to the proportion of colors (RGB values and the like) used in the painting, for example, it is possible to cause a visitor of the museum to experience the taste of "Mona Lisa", the taste of "Last Supper", and the like. By designing the electrical condition data corresponding to the viewing, listening, touching, and sniffing experiences in this manner, new entertainment can be provided.

### (10) Application to online tasting

By designing and accumulating electrical condition data that reproduces the taste of food, a user can simulate the taste of food provided in a luxurious restaurant or the like. As a result, the user can determine in advance whether the provided dish matches his/her preference without going to the store.

### (11) Application to unpalatable taste masking of pharmaceutical product

In many cases, a pharmaceutical product exhibits an unpalatable taste such as a bitter taste due to the characteristics of its components, which is a factor that hinders continuous dosage for patients. By designing the electrical condition data that masks the unpalatable taste of a pharmaceutical product, and providing the electrical condition data to a patient so that the pharmaceutical product is ingested together therewith, the unpalatable taste that is a problem can be masked.

### (12) Application to past and future food experiences

By designing and adjusting the electrical condition data that reproduces the taste of food, for example, it is possible to simulate a dish eaten in ancient Greek, a dish eaten in the past such as the taste of mother's food, a dish expecting the world in 100 years, the taste of a product (for example, premium wine) that is already sold out, and the like. As a result, the repertoire of food is increased, and a user can enjoy the pleasure of food more, which leads to improvement of people's well-being. In addition, the electrical condition can also be applied to food education for children by reproducing dishes eaten well in history and traditional foods eaten in each country/region.

### (13) Application to non-fungible token (NFT)

The electrical condition data for reproducing and constructing the taste of a food generated by a company, an influencer, an individual, or the like is distributed as NFT, thereby realizing a new food experience that can be experienced only by a person who properly purchases the electrical condition data. That is, trade of taste data is realized. For example, the electrical condition data suitable for a specific soup can be purchased by a limited number of 100 persons, and only those who purchase the electrical condition data can enjoy the soup while applying electricity.

### (14) Application to metaverse

The electrical condition data reproducing and constructing the taste of the food can realize more realistic eating experience at the time of having a meal on the metaverse. Even in a state of wearing a VR/AR/MR device, it is possible to have a delicious meal experience. In addition, for example, it is possible to increase the reality of imaginary foods (a cake in which lightning continues to flow, a curry with molten rock, a meat dish utilizing fallen demon, and the like) that can be eaten only on the metaverse.

### INDUSTRIAL APPLICABILITY

The present invention is useful in various industries such as, for example, the food industry, the food and drink industry, the medical industry, and the entertainment industry.

### EXPLANATIONS OF LETTERS OR NUMERALS

- 10: ELECTRICAL CONDITION DATA MANAGEMENT APPARATUS
- 102: CONTROL UNIT
- 102a: ACQUISITION UNIT
- 102b: SELECTION UNIT
- 102c: DETERMINATION UNIT
- 102d: READING UNIT
- 102e: READING-OUT UNIT
- 102f: FIRST TRANSMISSION UNIT
- 102g: INPUT UNIT
- 102h: REFLECTION UNIT
- 102i: GENERATION UNIT
- 102j: REGISTRATION UNIT
- 102k: SECOND TRANSMISSION UNIT
- 104: STORAGE UNIT
- 106: FIRST COMMUNICATION UNIT
- 108: SECOND COMMUNICATION UNIT
- 110: INPUT UNIT
- 112: OUTPUT UNIT
- 114: IMAGING UNIT

## Claims

1. An electrical condition data management apparatus comprising: a control unit; a storage unit; and a communication unit, wherein
the storage unit stores electrical condition data that changes a feeling of taste, and
the control unit comprising:
a reading-out unit that reads out the electrical condition data from the storage unit;
a first transmission unit that transmits the electrical condition data read out by the reading-out unit to an instrument including a communication unit, the instrument being capable of generating electricity corresponding to the electrical condition data received via the communication unit and causing the generated electricity to flow through a human body;
an input unit that inputs an impression of a taste held by a user in a state where the electricity corresponding to the electrical condition data transmitted by the first transmission unit flows through the user via the instrument; and
a reflection unit that reflects the impression input by the input unit in the electrical condition data stored in the storage unit.

2. The electrical condition data management apparatus according to claim 1, wherein
the electrical condition data is determined based on biological data.

3. The electrical condition data management apparatus according to claim 2, wherein
the biological data is activation data of an *in vivo* protein used when an organism feels the taste.

4. The electrical condition data management apparatus according to claim 3, wherein
the *in vivo* protein is a receptor or an ion channel.

5. The electrical condition data management apparatus according to claim 3 or 4, wherein
a numerical value of the electrical condition data is a numerical value corresponding to an intensity of the electrical condition data required to activate, even when a certain substance is not provided to the *in vivo* protein, the *in vivo* protein to the same extent as when the certain substance is provided to the *in vivo* protein.

6. The electrical condition data management apparatus according to any one of claims 1 to 5, wherein
the storage unit further stores at least one of personal information on the user, instrument information on the instrument, food information on food, and environmental information on an intake environment, and
the reflection unit further reflects the at least one information in the electrical condition data stored in the storage unit.

7. The electrical condition data management apparatus according to claim 6, wherein
the reflection unit inputs the electrical condition data after the at least one information corresponding to the user and the impression are reflected to a machine learning model that outputs, when the at least one information and the electrical condition data are input to the reflection unit, a congeniality degree between the at least one information and the electrical condition data, and determines, when the machine learning model outputs a result indicating that the congeniality degree therebetween is good, that the electrical condition data after the impression is reflected also reflect the information.

8. The electrical condition data management apparatus according to any one of claims 1 to 7, wherein
the control unit further comprises: a generation unit that generates the electrical condition data; and a registration unit that registers, in the storage unit, the electrical condition data generated by the generation unit.

9. The electrical condition data management apparatus according to claim 8, wherein
the storage unit further stores a plurality of templates related to the electrical condition data, and
the generation unit causes the user to generate the electrical condition data by causing the user to select a desired template from among the templates and causing the user to rewrite the selected template.

10. The electrical condition data management apparatus according to any one of claims 1 to 9, wherein
the control unit further comprises a second transmission unit that transmits the electrical condition data to another electrical condition data management apparatus.

11. The electrical condition data management apparatus according to any one of claims 1 to 10, wherein
a plurality of pieces of the electrical condition data are stored in the storage unit,
the control unit further comprises an acquisition unit that acquires preference information of the user, and a determination unit that determines, based on the preference information acquired by the acquisition unit, the electrical condition data matching a preference of the user from among the pieces of electrical condition data stored in the storage unit, and
the reading-out unit reads out, from the storage unit, the electrical condition data determined by the determination unit.

12. The electrical condition data management apparatus according to any one of claims 1 to 11, wherein
the storage unit further stores classification data including a plurality of classifications of the food,
the electrical condition data in the storage unit is stored in association with the classification of the food,
the control unit further includes a selection unit that causes the user to select the classification of the food,
the reading-out unit reads out, from the storage unit, the electrical condition data associated with the classification selected by the selection unit, and
the input unit inputs the impression of the taste when the user ingests an actual food corresponding to the selected classification in the state.

13. The electrical condition data management apparatus according to any one of claims 1 to 12, further comprising
an imaging unit, wherein
the electrical condition data in the storage unit is stored in association with the classification of the food,
the control unit includes a reading unit that reads a figure pattern from an image obtained by imaging, by the imaging unit, a subject on which the figure pattern in which the classification of the food is recorded is displayed and from which the classification of the food is visually recognizable,
the reading-out unit reads out, from the storage unit, the electrical condition data associated with the classification recorded in the figure pattern read by the reading unit, and
the input unit inputs the impression of the taste when the user ingests an actual food corresponding to the classification visually recognized from the subject in the state.

14. The electrical condition data management apparatus according to any one of claims 1 to 13, wherein
the storage unit further stores expression data including a plurality of expressions related to the taste, and
the input unit causes the user to select the appropriate expression from among the expressions.

15. The electrical condition data management apparatus according to any one of claims 1 to 14, wherein
the electrical condition data changes the feeling of the taste.

16. The electrical condition data management apparatus according to any one of claims 1 to 15, wherein
the electrical condition data includes at least one of a numerical value of an intensity of the electricity, a numerical value of a frequency of the electricity, and a numerical value of a duty ratio.

17. A program for causing a control unit of an electrical condition data management apparatus including the control unit, a storage unit that stores electrical condition data that changes a feeling of a taste, and a communication unit to function as:
a reading-out unit that reads out the electrical condition data from the storage unit;
a first transmission unit that transmits the electrical condition data read out by the reading-out unit to an instrument including a communication unit, the instrument being capable of generating electricity corresponding to the electrical condition data received via the communication unit and causing the generated electricity to flow through a human body;
an input unit that inputs an impression of a taste held by a user in a state where the electricity corresponding to the electrical condition data transmitted by the first transmission unit flows through the user via the instrument; and
a reflection unit that reflects the impression input by the input unit in the electrical condition data stored in the storage unit.

18. A method executed by a control unit of an electrical condition data management apparatus including the control unit, a storage unit that stores electrical condition data that changes a feeling of a taste, and a communication unit, the method comprising:
a step of reading out the electrical condition data from the storage unit;
a step of transmitting the read electrical condition data to an instrument including a communication unit, the instrument being capable of generating electricity corresponding to the electrical condition data received via the communication unit and causing the generated electricity to flow through a human body;
a step of inputting an impression of a taste held by a user in a state where the electricity corresponding to the transmitted electrical condition data flows through the user via the instrument; and
a step of reflecting the input impression in the electrical condition data stored in the storage unit.
